# EUROPEAN PATENT APPLICATION

(11) **EP 2 666 409 A1**
(43) Date of publication of application: **27.11.2013**
(21) Application number: 12196459.7
(22) Date of filing: 11.12.2012
(51) Int. Cl.: A61B 5/021, A61B 5/00

(54) **Measurement devices for bio-signals**

(30) Priority: 21.05.2012 TW 101117962
(71) Applicant: MedSense Inc., 30273 Zhubei City, Hsinchu County (TW)
(72) Inventor: Chiu, Benjamin, 302 Zhubei City, Hsinchu County (TW)
(74) Representative: 2K Patentanwälte Blasberg Kewitz & Reichel

(57) **Abstract**

A measurement device (1) is provided. A sensor (11) senses a vessel pulse waveform of a specific region of an object to generate a vessel pulse signal in a measurement mode. In the measurement mode, a first electrode (12) generates a first potential signal, and the second electrode (13) generates a second potential signal. A first analog front-end circuit (22) digitizes the vessel pulse signal to generate a digital vessel pulse signal in the measurement mode. In the measurement mode, a second analog front-end circuit (23) obtains an electrocardiogram signal according to the first and second potential signals and digitizes the electrocardiogram signal. A memory (24) stores the digital vessel pulse signal and the electrocardiogram signal. A processor determines a polarity of the electrocardiogram signal in the measurement mode to indicate that the specific region is on a left or right part of a body of the object.

## Description

### CROSS REFERENCE TO RELATED APPLICATIONS

This Application claims priority of Taiwan Patent Application No. 101117962, filed on May 21, 2012, the entirety of which is incorporated by reference herein.

### BACKGROUND OF THE INVENTION

### FIELD OF THE INVENTION

The invention relates to a measurement device, and more particularly to a device for measuring a vessel pulse signal and an electrocardiogram signal of an object, thereby obtaining a blood pressure value and a pulse wave velocity to serve as an index of risk possibility of arterial stiffness.

### DESCRIPTION OF THE RELATED ART

With aging societies, more and more burden is being placed on hospital resources. Moreover, cardiovascular diseases are increasing, as people age and stress increases for modern day living. For example, high blood pressure and arterial stiffness are normal symptoms of cardiovascular diseases. Thus, arterial stiffness and bio-signal self-measurement devices becomes an important target of development in the healthcare industry. Through bio-signal self-measurement manners, patients can monitor their own physiology status anytime, to relieve strain on hospital resources and provide needed medical attention to patients.

Currently, domestic blood pressure meters are universal. However, detection apparatuses for arterial stiffness are very expensive and not easily operated by users. For example, in the detection apparatus provided by OMRON, the vessel pulse signals of the extremity of the users have to be measured to obtain a pulse wave velocity (PWV) which serves as an index of risk possibility of arterial stiffness. Thus, the detection apparatus provided by OMRON is not carried easily by the users and, thus, not universally used. Some prior arts disclose that the electrocardiogram signal and the vessel pulse signal are used to obtain the pulse wave velocity. However, the users have to set the position information of the measured region, which is not convenient for the users. Moreover, measurement error may occur when the users set the wrong position information. Thus, the present invention provides a device which is carried and operated easily and also used to obtain the pulse wave velocity.

### BRIEF SUMMARY OF THE INVENTION

An exemplary embodiment of a measurement device is provided. The measurement device measures a vessel pulse signal of a specific region of an object in a measurement mode. The measurement device comprises a case, a sensor, a first electrode, a second electrode, a first analog front-end circuit, a second analog front-end circuit, a memory, and a processor. The case has a first side and a second side opposite to the first side. In the measurement mode, the first side faces the specific region of the object. The sensor is disposed on the first side. The sensor senses a vessel pulse waveform of the specific region to generate the vessel pulse signal in the measurement mode. The first electrode is disposed on the first side. The first electrode generates a first potential signal in the measurement mode. The second electrode generates a second potential signal in the measurement mode. The first analog front-end circuit is coupled to the sensor. The first analog front-end circuit digitizes the vessel pulse signal to generate a digital vessel pulse signal in the measurement mode. The second analog front-end circuit is coupled to the first electrode and the second electrode. In the measurement mode, the second analog front-end circuit obtains an electrocardiogram signal according to the first potential signal and the second potential signal and digitizes the electrocardiogram signal. The memory stores the digital vessel pulse signal and the electrocardiogram signal. The processor determines a polarity of the electrocardiogram signal in the measurement mode to indicate that the specific region is on a left part or a right part of a body of the object.

A detailed description is given in the following embodiments with reference to the accompanying drawings.

### BRIEF DESCRIPTION OF THE DRAWINGS

The invention can be more fully understood by reading the subsequent detailed description and examples with references made to the accompanying drawings, wherein:

FIG. 1A shows the appearance of an exemplary embodiment of a measurement device;

FIG. 1B shows a side view of the measurement device in FIG. 1A;

FIG. 2 is a schematic block diagram of the measurement device when an external sub-device is not connected to the measurement device;

FIG. 3 is a schematic block diagram of the measurement device when an external sub-device has been connected to the measurement device;

FIG. 4 shows a vessel pulse signal, an electrocardiogram signal, and an aorta blood pressure signal of an object;

FIG. 5 is a schematic block diagram of the measurement device when an external host has been connected to the measurement device; and

FIG. 6 shows another exemplary embodiment of a measurement device.

### DETAILED DESCRIPTION OF THE INVENTION

The following description is of the best-contemplated mode of carrying out the invention. This description is made for the purpose of illustrating the general principles of the invention and should not be taken in a limiting sense. The scope of the invention is best determined by reference to the appended claims.

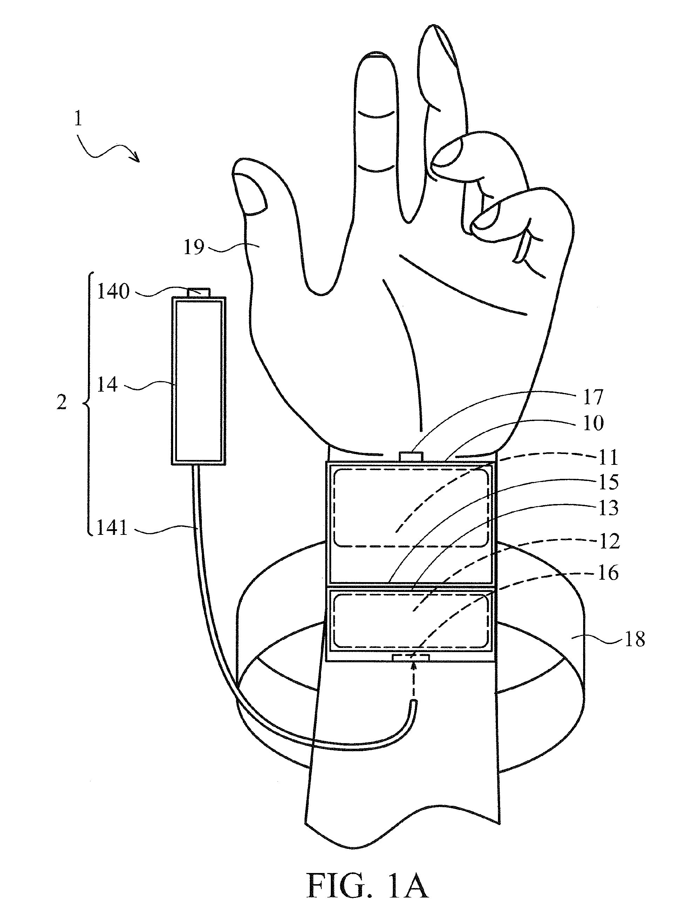

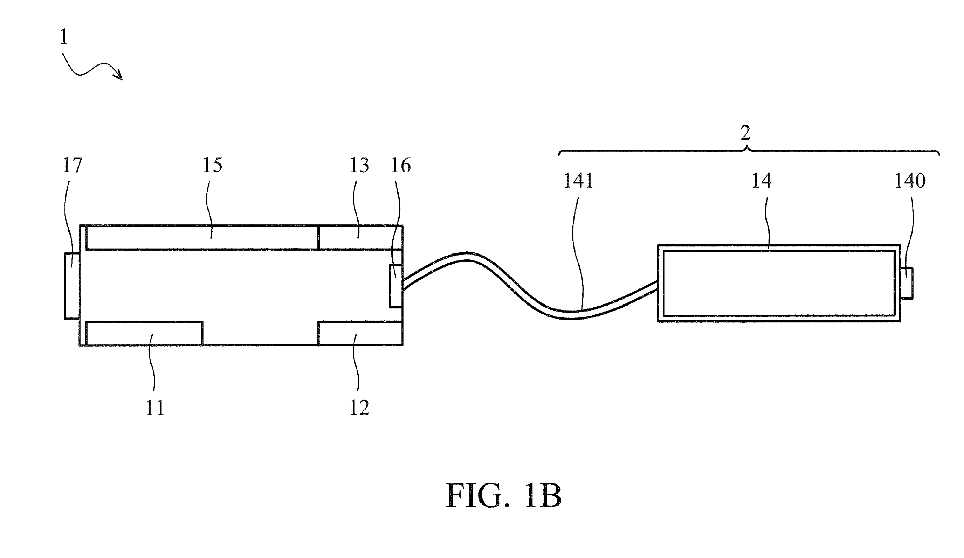

FIG. 1A shows the appearance of an exemplary embodiment of a measurement device, and FIG. 1B shows a side view of the measurement device in FIG. 1A. Referring to FIGS. 1A and 1B, when viewing the appearance of a measurement device 1, the measurement device 1 comprises an external sub-device 2, a case 10, a sensor 11, electrodes 12 and 13, a displayer 15, a connection port 16, a power switch 17, and a bracelet 18 (not shown in FIG. 1B). The case 10 is disposed on the bracelet 18. The case 10 has an inner side and an outer side. When the measurement device 1 has been placed on a specific region of an object (such as the wrist or leg) to measure bio-signals, the measurement device 1 is tied onto the specific region of the object through the bracelet 18, and, at this time, the inner side of the case 10 faces the specific region of the object. The sensor 11 and the electrode 12 are disposed on the inner side of the case 10, while the electrode 13 is disposed on the outer side of the case 10. The connection port 16 is disposed on one edge of the case 10, such as the upper edge. The power switch 17 is disposed on another edge of the case 10, such as the lower edge. In the embodiment of FIGs. 1A and 1B, the positions of the connection port 16 and the power switch 17 on the case 10 are given as an example. In other embodiments, the connection port 16 and the power switch 16 can be disposed on the same edge of the case 10.

The external sub-device 2 is selectively connected to the connection port 16. Referring to FIGs. 1A and 1B, the external sub-device 2 comprises an electrode 14, a control button 140, and a transmission line 141. When it is desired to use the external sub-device 2, the transmission line 141 has to be connected to the connection port 16. In an embodiment, the connection port 16 is a port conforming to the USB standard.

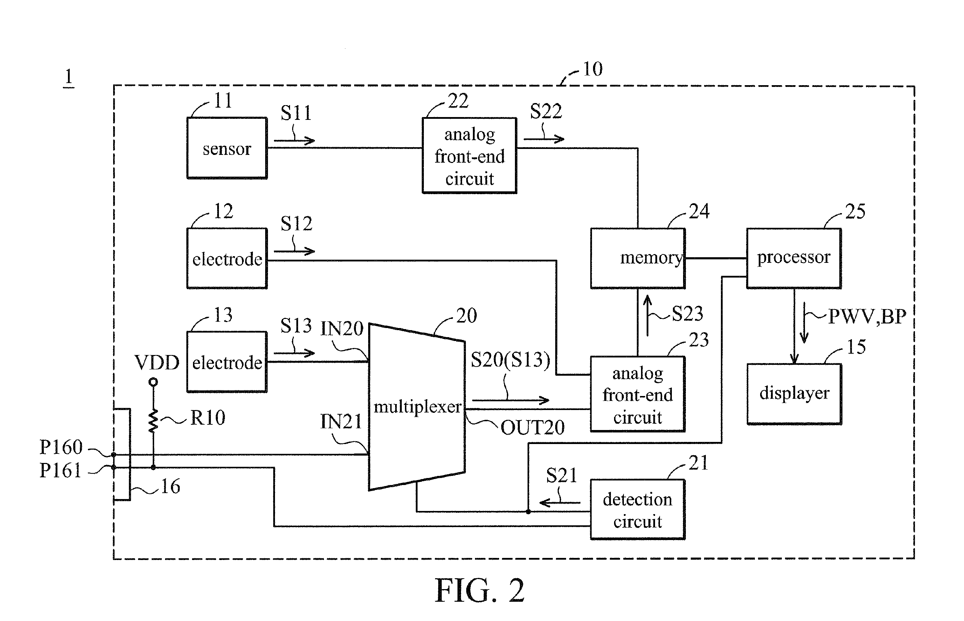

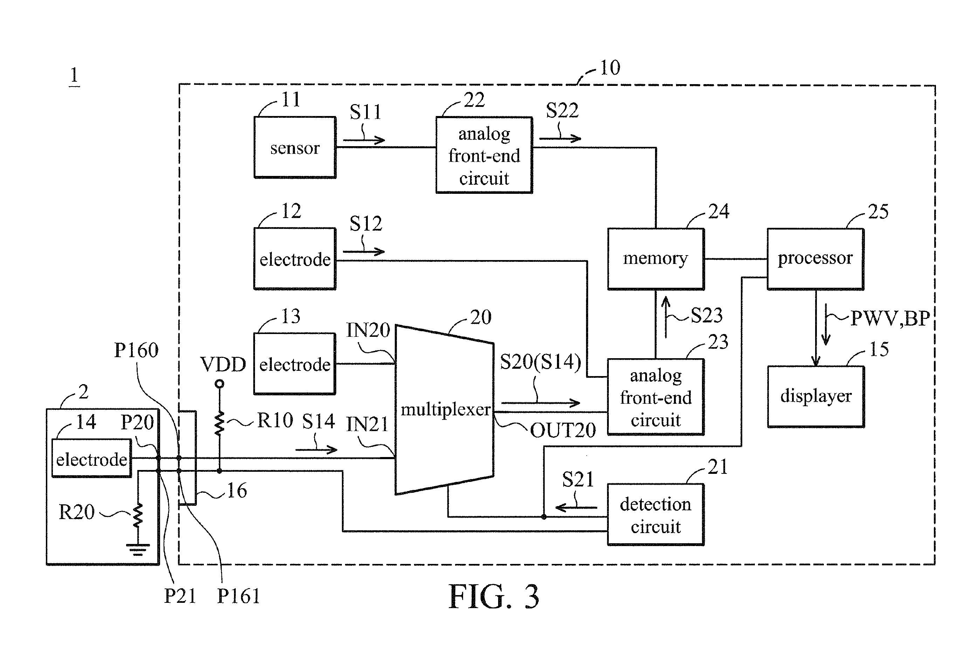

When the power switch 17 of the measurement device 1 is turned on and the measurement device 1 is tied onto the specific region of the object, the measurement device 1 enters a measurement mode. Each of the sensor 11 and the electrodes 12-14 is used o contact a region of the body of the object. The sensor 11 is used to sense a vessel pulse waveform of the contacted region of the object to generate a vessel pulse signal S11. The vessel pulse signal S11 is digitized and then stored in a memory 24 (shown in FIG. 2). A processor 25 (shown in FIG. 2) obtains blood pressure value BP according to the vessel pulse signal S11. In the embodiment, the sensor 11 can be a pressure sensor or an optical sensor. Each of the electrodes 12-14 is used to sense an electrical signal on the contacted region of the object to generate a corresponding potential signal S12, S13, or S14 (shown in FIGs. 2 and 3). In the embodiment, the electrodes 12-14 touch the skin of the contacted region of the object.

When it is desired to measure bio-signals of the specific region of the object, the measurement device 1 is tied onto the specific region of the body of the object through the bracelet 18, such as an upper or lower limb of the left part of the body of the object (e.g. the left wrist or leg) or an upper or lower limb of the right part of the body of the object (e.g. the right wrist or leg). For example, when the measurement device 1 is tied onto an upper limb of the left part of the body of the object (left wrist) through the bracelet 18, the sensor 11 and the electrode 12 which are disposed on the inner side of the case 10 contact the upper limb. At this time, the electrode 13 which is disposed on the outer side of the case 10 contacts the right hand of the object (the right fingers contact the electrode 13). In this case, the external sub-device 2 is not connected to the connection port 16. In another case, when the measurement device 1 is tied onto a lower limb of the left part of the body of the object (left leg) through the bracelet 18, the sensor 11 and the electrode 12 which are disposed on the inner side of the case 10 contact the lower limb. At this time, the external sub-device 2 has been connected to the connection port 16, and the electrode 14 contacts the right hand of the object (such as that the right hand grips the electrode 14). Moreover, at this time, the electrode 13 does not contact the object, and, thus, the electrode 13 is inactive.

The detailed structure and operation of the measurement device 1 of the embodiment will be described in the following.

FIG. 2 shows a block diagram of the measurement device 1. The case 10, the sensor 11, the electrodes 12 and 13, the displayer 15, the connection port 16, and the power switch 17 shown in FIG. 2 are the devices or elements which are observed from the appearance of a measurement device 1. Referring to FIG. 2, the measurement device 1 further comprises a multiplexer 20, a detection circuit 21, an analog front-end circuits 22 and 23, a memory 24, and a processor 25 which are disposed in the case 10. FIG. 2 does not show the power switch 17 and the bracelet 18, however, they are shown in FIG. 1A.

The multiplexer 20 has two input terminals IN20 and IN21 and an output terminal OUT20. The input terminal IN20 is coupled to the electrode 13, and the input terminal IN21 is coupled to a pin P160 of the connection port 16 (the connection of the pins of the connection port 16 will be described in the following). The multiplexer 20 is controlled by a detection signal S21 to selectively transmit a signal at the input terminal IN20 or IN21 to the output terminal OUT20 to serve as a potential signal S20. The detection signal S21 is generated by the detection circuit 21. In the measurement mode, the detection circuit 21 detects whether the external sub-device 2 has been connected to the connection port 16 and generates the detection signal S21 according to the detection result. When the detection circuit 21 detects that the external sub-device 2 is not connected to the connection port 16, the multiplexer 20 transmits the potential signal S13 from the electrode 13 to the output terminal OUT20 according to the detection signal S21 to serve as the potential signal S20. For example, referring to FIG. 2, in the case where the external sub-device 2 is not connected to the connection port 16 in the measurement mode, the measurement device 1 is tied onto an upper limb of the left part of the body of the object (such as the left wrist, referred to the specific region), and the sensor 11 and the electrode 12 which are disposed on the inner side of the case 10 contact the upper limb to generate the vessel pulse signal S11 and the potential signal S12, respectively. At this time, the electrode 13 which is disposed on the outer side of the case 10 contacts the right fingers of the object to generate the potential signal S13. The multiplexer 20 transmits the potential signal S13 from the electrode 13 to the output terminal OUT20 according to the detection signal S21 to serve as the potential signal S20.

Referring to FIG. 3, when the detection circuit 21 detects that the external sub-device 2 has been connected to the connection port 16, the multiplexer 20 transmits the potential signal S14 from the electrode 14 according to the detection signal S21 to serve as the potential signal S20. For example, in the case where the external sub-device 2 has been connected to the connection port 21, the measurement device 1 is tied onto a lower limb of the left part of the body of the object (such as the left leg, referred to the specific region), and the sensor 11 and the electrode 12 which are disposed on the inner side of the case 10 contact the lower limb to generate the vessel pulse signal S11 and the potential signal S12, respectively. At this time, the electrode 14 contacts a right limb of the object (such as that the right hand grips the electrode 14) to generate the potential signal S14. The multiplexer 20 transmits the potential signal S14 from the electrode 14 to the output terminal OUT20 according to the detection signal S21 to serve as the potential signal S20.

The analog front-end circuit 22 receives the vessel pulse signal S11 from the sensor 11 and performs a digitization operation to the vessel pulse signal S11, such as an amplifying, filtering, and analog-digital converting operation, to generate a digital vessel pulse signal S22. The digital vessel pulse signal S22 is transmitted to the memory 24 and stored in the memory 24. The analog front-end circuit 23 receives the potential signal S12 from the electrode 12 and the potential signal S20 from the multiplexer 20 and generates an electrocardiogram signal S23 according to the potential difference between the potential signals S12 and S20. The analog front-end circuit 23 also performs a digitalization operation to the electrocardiogram signal S23, such as an amplifying, filtering, and analog-digital converting operation. The digitalized electrocardiogram signal S23 is transmitted to the memory 24 and stored in the memory 24.

The processor 26 reads the digital vessel pulse signal S22 and the electrocardiogram signal S23 from the memory 24. In the measurement mode, the processor 25 first determines whether the electrocardiogram signal S23 is at a periodically stable state. In the embodiment, the processor 25 may determine whether the QRS complex repeatedly occurs in the electrocardiogram signal S23 to determine whether the electrocardiogram signal S23 is at the periodically stable state. When the QRS complex repeatedly occurs in the electrocardiogram signal S23, the processor 25 determines that the electrocardiogram signal S23 is at the periodically stable state.

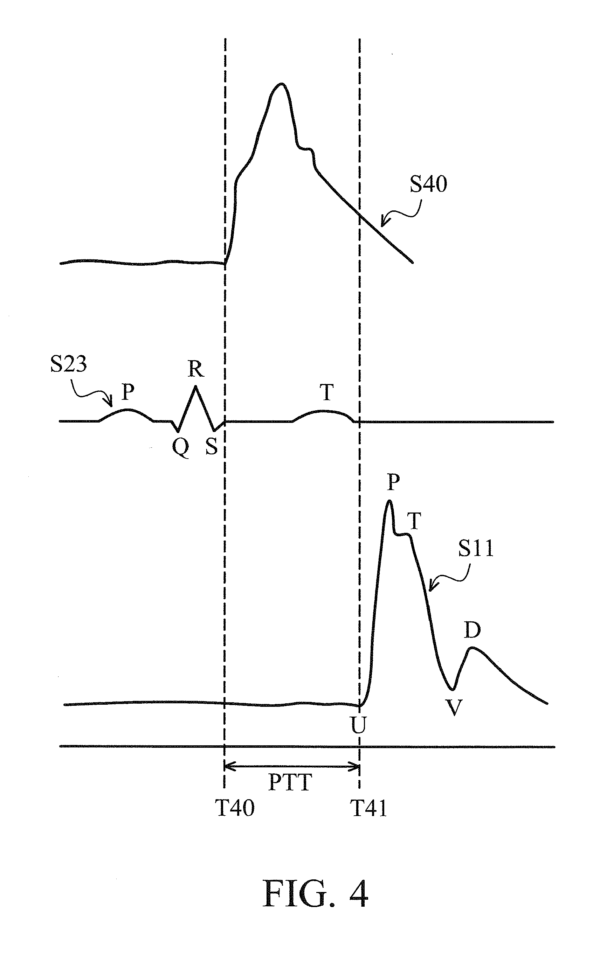

When the processor 25 determines that the electrocardiogram signal S23 is at the periodically stable state, the processor 25 starts to retrieve a reference time point on the electrocardiogram signal S23 and a reference time point on the vessel pulse signal S11. The processor 25 calculates the difference between the two reference time points to obtain a pulse transmission time (PTT) which indicates the time period when the pressure wave of the blood pressure is output to the specific region of the object from the heart of the object. FIG. 4 shows the vessel pulse signal S11, the electrocardiogram signal S23, and an aorta blood pressure signal S40 of the object. When the aortic valve of the heart of the object is open, the blood flows to the aorta from the heart. At this time, the blood pressure of the aorta starts rising. The blood pressure of the limbs starts rising after a delayed time period. Referring to FIG. 4, the signal S40 starts rising at a time point T40. Thus, it can be determined that at the time point T40, the blood starts flowing to the aorta from the heart. Comparing the signal S40 and the electrocardiogram signal S23, the time point T40 is the time point when a second differentiation value of the electrocardiogram signal S23, which is obtained when the a second differentiation is applied to the electrocardiogram signal S23 by the processor 25, begins to be 0 after the QRS complex of the electrocardiogram signal S23 occurs. Thus, in the embodiment, the processor 25 applies a second differentiation to the electrocardiogram signal S23 and retrieves the time point when the obtained second differentiation value of the electrocardiogram signal S23 begins to be 0 after the QRS complex of the electrocardiogram signal S23 occurs to serve as the reference time point T40.

Moreover, the processor 25 retrieves the time point when a rising waveform starts appearing on the vessel pulse signal S11 to serve as the other reference point. The rising waveform indicates that the blood pressure of the specific region rises. In the embodiment, the processor 25 applies a first differentiation to the vessel pulse signal S11 and retrieves the time point when the first differentiation value of the vessel pulse signal S11 near the U point of the vessel pulse signal S11 is equal to 0 to serve as the reference time point T41. The processor 25 calculates the difference between the reference time points T41 and T40 to obtain the pulse transmission time.

Moreover, when the processor 25 determines that the electrocardiogram signal S23 is at the periodically stable state, the process 25 also starts detecting the polarity of the electrocardiogram signal S23 to determine that the measurement device 1 has been placed on the left or right part of the body of the object (that is to determine that the specific region is on the left or right part of the body of the object). For example, when the electrocardiogram signal S23 has a positive polarity, the process 23 determines that the measurement device 1 has been placed on the left part of the body of the object and when the electrocardiogram signal S23 has a negative polarity, the process 23 determines that the measurement device 1 has been placed on the right part of the body of the object. Further, the processor 25 also receives the detection signal S21 from the detection circuit 21. As the above describes, the detection signal S21 indicates whether the external sub-device 2 has been connected to the connection port 16. Since the external sub-device 2 has been connected to the connection port 16 only when the measurement device 1 has been placed on a lower limb of the object, the detection signal S21 can indicate that the measurement device 1 has been placed on an upper or lower limb of the object (that is, the specific region is on an upper or lower limb of the object). Accordingly, when the processor 25 determines that the electrocardiogram signal S23 is at the periodically stable state, the processor 25 confirms that the specific region is on an upper limb of the left part of the body of the object (such as the left wrist), a lower limb of the left part of the body of the object (such as the left leg), an upper limb of the right part of the body of the object (such as the right wrist), or a lower limb of the right part of the body of the object (such as the right leg) according to the polarity of the electrocardiogram signal S23 and the detection signal S21.

When the processor 25 completes the confirmation of the specific region, the processor 25 obtains the distance between the specific region and the heart of the object by applying the height of the object into the stand body proportion equation to serve as a measurement distance. The height of the object is set in the measurement device 1 in advance by the object. The processor 25 then divides the measurement distance by the pulse transmission time to obtain a pulse wave velocity PWV. The processor 25 may transmit the pulse wave velocity PWV to the displayer 15, and the pulse wave velocity PWV is shown on the displayer 15. The pulse wave velocity PWV is an important index of risk possibility of arterial stiffness. Thus, the object can determine a risk possibility of arterial stiffness according to the pulse wave velocity PWV. Moreover, the displayer 15 can also display operation conditions of the measurement device 1, such as the remaining power of the battery.

As the above describes, the vessel pulse signal S11 indicates the blood pressure. Thus, in an embodiment, when the processor 25 determines that the electrocardiogram signal S23 is at the periodically stable state, the processor 25 further determines the blood pressure value BP of the object according to the amplitude of the vessel pulse signal S11. The processor 25 transmits the determined blood pressure value BP to the displayer 15, and the determined blood pressure value BP is shown in the displayer 15. Since the processor 25 confirms the position of the specific region according to the polarity of the electrocardiogram signal S23 and the detection signal S21, the object or medical employees can be aware of which region of the body of the object that the blood pressure value blood is being measured from, thus, the object can be diagnosed by the medical employees accurately.

In an embodiment, the processor 25 comprises a database which stores a plurality of pulse wave velocity reference values and a plurality of risk possibility reference values of arterial stiffness, and each pulse wave velocity reference value corresponds to one risk possibility reference value of arterial stiffness. After the processor 25 obtains the pulse wave velocity PWV, the processor 25 searches the database according to the value of the pulse wave velocity PWV to obtain one corresponding risk possibility reference value of arterial stiffness. The processor 25 transmits the obtained risk possibility reference value of arterial stiffness to the displayer 15 for displaying, such that the object is aware of a risk possibility value of arterial stiffness according to the value shown on the displayer 15. The database also stores a plurality of amplitude reference values and a plurality of blood pressure reference values. After the processor 25 obtains the amplitude of the vessel pulse signal S11, the processor 25 searches the database according to the amplitude to obtain one blood pressure value. The blood pressure value comprises a systolic pressure value and/or a diastolic pressure value. The risk of the cardiovascular disease can be estimated holistically.

In the above embodiment, in the measurement mode, when the measurement device 1 is tied onto a lower limb of the object, the external sub-device 2 is required to be connected to the connection port 16. In other modes, the connection port 16 can be connected with other devices. In an embodiment, when the measurement device 1 is in a charging mode, an external power source has been connected to the connection port 16 to charge the measurement device 1. In another embodiment, when the measurement device 1 is in a data transmission mode, an external host has been connected to the connection port 16, such that measurement data stored in the memory 24 of the measurement device 1 can be transmitted to the external host, wherein the measurement data is obtained when the measurement device 1 operates in the measurement mode once or many times. The measurement data comprises the date and time when the measurement data is obtained, the digital vessel pulse signal S22, and the electrocardiogram signal S23.

In the embodiment, the detection circuit 21 is used to detect whether the external sub-device 2 has been connected to the connection port 16. In an embodiment, the detection circuit 21 detects whether the external sub-device 2 has been connected to the connection port 16 by detecting the voltage at a specific pin of the connection port 16. For example, the connection port 16 has at least two pins (excluding the power pins VDD and GND), and the transmission line 141 of the external sub-device 2 has pins with the same number as the pins of the of the connection port 16. As shown in FIGs. 2 and 3, the connection port 16 has two pins P160 and P161. The input terminal IN21 of the multiplexer 20 is coupled to the pin P160. The measurement device 1 comprises a pull-up resistor R10 which is coupled between a supplying voltage VDD and the pin P161. Moreover, the detection circuit 21 is also coupled to the pin P161. Referring to FIG. 3, the external sub-device 2 also has two pins P20 and P21. The electrode 14 of the external sub-device 2 is coupled to the pin P20. The external sub-device 2 comprises a pull-down resistor R20 which is coupled between the pin P21 and the ground. When the external sub-device 2 has been connected to the connection port 16 in the measurement mode, the pins P20 and P21 of the external dub-device 2 are coupled to the pins P160 and P161 of the connection port 16, respectively. At this time, the voltage is obtained from the pin P161 according to the resistance values of the pull-up resistor R10 and the pull-down resistor R20. When the detection circuit 21 detects that the pin P161 has the voltage, the detection circuit 21 determines that the external sub-device 2 has been connected to the connection port 16 through the transmission line 161, and the potential signal S14 is obtained from the pin P160 and transmitted to the input terminal IN21 of the multiplexer 20.

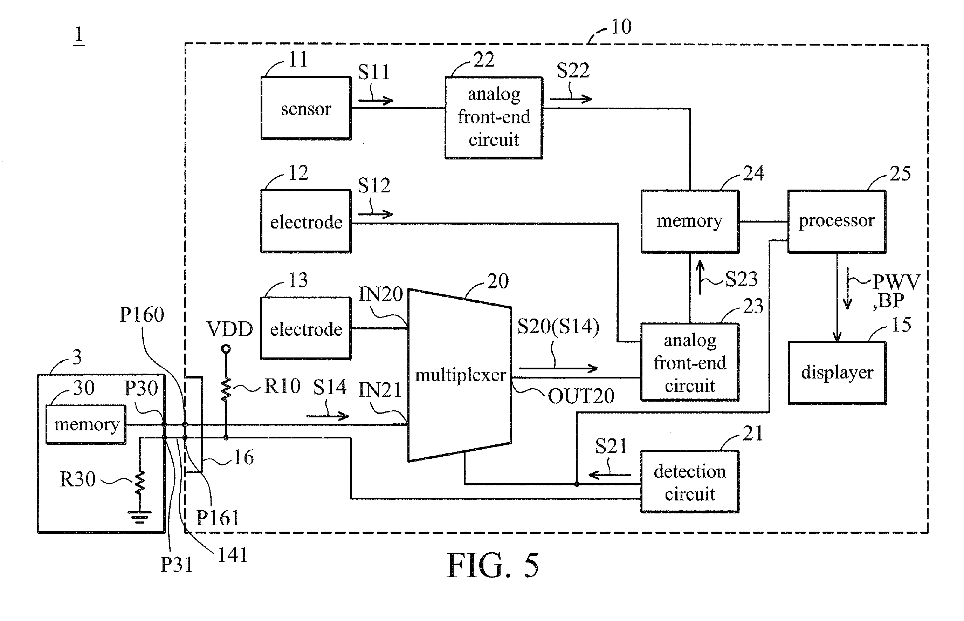

Referring to FIG. 5, in the data transmission mode, an external host 3 has been connected to the connection port 16. The external host 3 also has pins with the same number as the pins of the of the connection port 16. As shown in FIG. 5, the external host has two pins P30 and P31. The external host 3 comprises a memory 30 and a pull-down resistor R30. The memory 30 is coupled to the pin P30, and the pull-down resistor R30 is coupled between the pin P31 and the ground. In the data transmission mode, another voltage is obtained from the pin P161 according to the resistance values of the pull-up resistor R10 and the pull-down resistor R30 of the external host 3. When the detection circuit 21 detects that the pin P161 has the voltage, the detection circuit 21 determines that the external host 3 has been connected to the connection port 16. According to the above detection operation, the detection circuit 21 can determine that the device connected to the connection port 16 is the external sub-device 2 or the external host 3 according to the voltage at the pin P161.

In another embodiment, a mechanical switch is disposed by the side of the connection port 16. When the external sub-device 2 has been connected to the connection port 16 through the transmission line 141, the mechanical switch is touched and then turned on. At this time, the detection device 21 receives a turned-on signal from the mechanical signal to determine that the external sub-device 2 has been connected to the connection port 16.

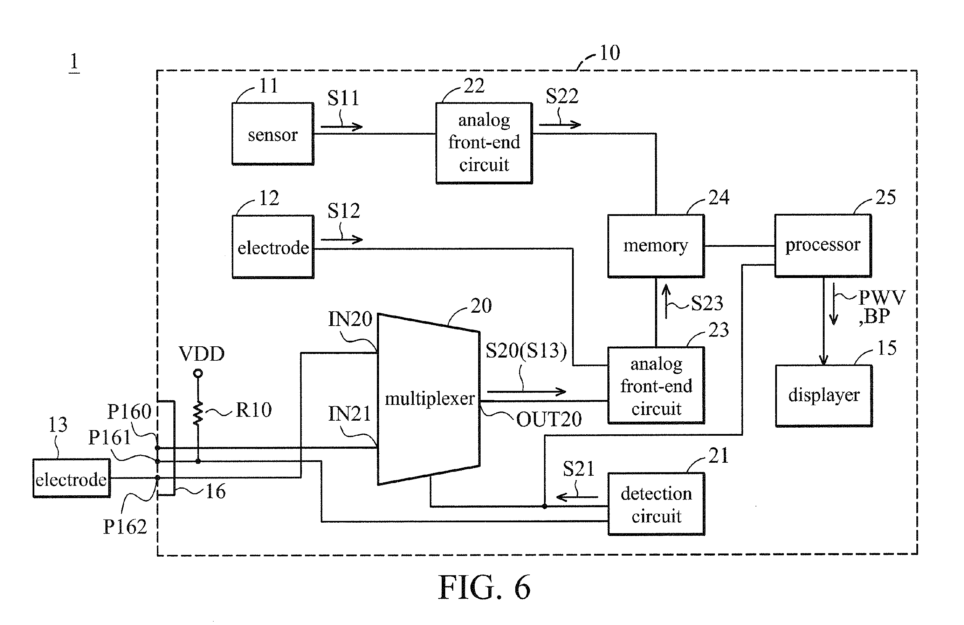

In the above embodiment, the electrode 13 is disposed on the outer side of the case 10. In another embodiment, the electrode 13 may be coupled to the case 10 through the connection port 16. As shown in FIG. 6, the connection port 16 further has a pin P162. The electrode 13 is coupled to the input terminal IN20 of the multiplexer 20 through the pin P162.
In summary of the above, there is provided a measurement device 1. A sensor 11 senses a vessel pulse waveform of a specific region of an object to generate a vessel pulse signal in a measurement mode. In the measurement mode, a first electrode 12 generates a first potential signal, and the second electrode 13 generates a second potential signal. A first analog front-end circuit 22 digitizes the vessel pulse signal to generate a digital vessel pulse signal in the measurement mode. In the measurement mode, a second analog front-end circuit 23 obtains an electrocardiogram signal according to the first and second potential signals and digitizes the electrocardiogram signal. A memory 24 stores the digital vessel pulse signal and the electrocardiogram signal. A processor determines a polarity of the electrocardiogram signal in the measurement mode to indicate that the specific region is on a left or right part of a body of the object.

While the invention has been described by way of example and in terms of the preferred embodiments, it is to be understood that the invention is not limited to the disclosed embodiments. To the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A measurement device (1) for measuring a vessel pulse signal of a specific region of an object in a measurement mode, comprising:
a case (10) having a first side and a second side opposite to the first side, wherein in the measurement mode, the first side faces the specific region of the object;
a sensor (11), disposed on the first side, for sensing a vessel pulse waveform of the specific region to generate the vessel pulse signal in the measurement mode;
a first electrode (12), disposed on the first side, for generating a first potential signal in the measurement mode;
a second electrode (13) for generating a second potential signal in the measurement mode;
a first analog front-end circuit (22), coupled to the sensor (11), for digitizing the vessel pulse signal to generate a digital vessel pulse signal in the measurement mode;
a second analog front-end circuit (23), coupled to the first electrode (12) and the second electrode (13), for, in the measurement mode, obtaining an electrocardiogram signal according to the first potential signal and the second potential signal and digitizing the electrocardiogram signal;
a memory (24) for storing the digital vessel pulse signal and the electrocardiogram signal; and
a processor (25) for determining a polarity of the electrocardiogram signal in the measurement mode to indicate that the specific region is on a left part or a right part of a body of the object.

2. The measurement device as claimed in claim 1, wherein in the measurement mode, the first electrode (12) contacts an upper limb of one of the left part and the right part of the body of the object to generate the first potential, and the second electrode (13) contacts an upper limb of the other of the left part and the right part of the body of the object to generate the second potential signal.

3. The measurement device as claimed in claim 1 or 2, wherein the second electrode (13) is disposed on the second side of the case (10).

4. The measurement device as claimed in one of the preceding claims further comprising:
a connection port (16) disposed on the case and having a pin, wherein the second electrode (13) is connected to the pin.

5. The measurement device as claimed in one of the claims 1-3 further comprising:
a third electrode (14);
a connection port (16) disposed on the case and having a first pin, wherein in the measurement mode, the third electrode (14) is selectively connected to the first pin to generate a third potential signal;
a detection circuit (21) for detecting whether the third electrode has been connected to the first pin to generate a detection signal in the measurement mode to indicate that the specific region is on an upper limb or a lower limb of the object; and
a multiplexer (20) having a first input terminal coupled to the second electrode, a second input terminal coupled to the connection port, and an output terminal outputting a fourth potential signal and controlled by the detection signal,
wherein in the measurement mode, when the detection circuit (21) detects that the third electrode is not connected to the connection port (16), the multiplexer (20) selects the second potential signal from the second electrode to be transmitted to the output terminal to serve as the fourth potential signal,
wherein in the measurement mode, when the detection circuit (21) detects that the third electrode has been connected to the connection port (16), the multiplexer (20) selects the third potential signal from the third electrode to be transmitted to the output terminal to serve as the fourth potential signal, and
wherein in the measurement mode, the second analog front-end circuit (23) obtains the electrocardiogram signal according to a potential difference between the first potential signal and the second potential, and the processor (25) reconfirms that the specific region is on a left upper limb, a left lower limb, a right upper limb, or a right lower limb according to the detection signal and the electrocardiogram signal read from the memory.

6. The measurement device as claimed in claim 5,
wherein in the measurement mode, when the third electrode (14) is not connected to the connection port, the first electrode contacts an upper limb of one of the left part and the right part of the body of the object to generate the first potential, and the second electrode contacts an upper limb of the other of the left part and the right part of the body of the object to generate the second potential signal, and
wherein in the measurement mode, when the third electrode (14) has been connected to the connection port (16), the first electrode contacts a lower limb of one of the left part and the right part of the body of the object to generate the first potential, and the third electrode contacts an upper limb of the other of the left part and the right part of the body of the object to generate the third potential signal

7. The measurement device as claimed in one of the preceding claims,
wherein in the measurement mode, the processor (25) obtains a measurement distance between the specific region and the heart of the object,
wherein in the measurement mode, the processor (25) retrieves a first reference time point on the electrocardiogram signal and a second reference time point on the vessel pulse signal, and the second reference time point is later than the first reference time point, and
wherein in the measurement mode, the processor (25) calculates a difference between the second reference time point and the first reference time point to obtain a pulse transmission time and divides the measurement distance by the pulse transmission time to obtain a pulse wave velocity.

8. The measurement device as claimed in claim 7, wherein in the measurement mode, when the processor (25) determines that the electrocardiogram signal is at a periodically stable state, the processor (25) starts determining the polarity of the electrocardiogram signal, calculating the measurement distance, and calculating the pulse wave velocity.

9. The measurement device as claimed in claim 8, wherein when QRS complex repeatedly occurs in the electrocardiogram signal, the processor (25) determines that the electrocardiogram signal is at the periodically stable state.

10. The measurement device as claimed in claim 7 further comprising:
a displayer, disposed on the first side of the case, for displaying the pulse wave velocity and operation conditions of the measurement device.

11. The measurement device as claimed in one of the preceding claims, wherein the sensor (11) is a pressure sensor or an optical sensor.

12. The measurement device as claimed one of the preceding claims, wherein the processor (25) calculates a blood pressure value of the specific region according to the vessel pulse signal.

13. The measurement device as claimed in claim 12 further comprising:
a displayer (15), disposed on the first side of the case, for displaying the blood pressure value and operation conditions of the measurement device.

14. The measurement device as claimed in claim 12, wherein in the measurement mode, when the processor (25) determines that electrocardiogram signal is at a periodically stable state, the processor starts calculates the blood pressure value.

15. The measurement device as claimed in claim 14, wherein when QRS complex repeatedly occurs in the electrocardiogram signal, the processor (25) determines that the electrocardiogram signal is at the periodically stable state.
